# EUROPÄISCHE PATENTSCHRIFT

(11) **EP 0 732 892 B1**
(45) Veröffentlichungstag und Bekanntmachung des Hinweises auf die Patenterteilung: **09.09.1998**
(21) Anmeldenummer: 94928869.0
(22) Anmeldetag: 06.10.1994
(51) Int. Cl.: A61B 17/28, A61B 17/32

(54) **CHIRURGISCHES ROHRSCHAFTINSTRUMENT**
TUBULAR-HANDLED SURGICAL INSTRUMENT
INSTRUMENT CHIRURGICAL TUBULAIRE

(30) Priorität: 08.12.1993 DE 4341736
(43) Veröffentlichungstag der Anmeldung: 25.09.1996
(73) Patentinhaber: AESCULAP AG, 78532 Tuttlingen (DE)
(72) Erfinder: GIORDANO, Nicola, D-78056 Villingen-Schwenningen (DE); MORALES, Pedro, D-78532 Tuttlingen (DE); NESPER, Markus, D-78532 Tuttlingen (DE); TADDIA, Lino, D-78573 Wurmlingen (DE); WEISSHAUPT, Dieter, D-78194 Immendingen (DE)
(74) Vertreter: Böhme, Ulrich
(86) Internationale Anmeldenummer: EP9403308
(87) Internationale Veröffentlichungsnummer: WO9515720

(56) Entgegenhaltungen:
- EP-A- 0 513 471
- WO-A-91/05514
- GB-A- 2 140 735
- US-A- 4 944 741
- US-A- 5 152 779

## Beschreibung

Die Erfindung betrifft ein chirurgisches Rohrschaftinstrument mit zwei gelenkig miteinander verbundenen Griffbranchen, mit einem an einer Griffbranche gehaltenen Rohr, einem an dessen Ende gelagerten Werkzeug, einem im Rohr längsverschieblich gelagerten, stabförmigen Betätigungselement für das Werkzeug, das über ein zylindrisches oder kugeliges Ende gelenkig mit der anderen Griffbranche Verbunden ist, und mit einer lösbaren Verbindung zwischen Rohr und Betätigungselement einerseits und den beiden Griffbranchen andererseits, wobei das Rohr in eine rohrförmige Aufnahme an der einen Griffbranche einschiebbar und in dieser durch mindestens einen radial bewegbaren, in einer Endstellung mit dem Rohr einen Formschluß eingehenden Verriegelungskörper axial festlegbar ist und wobei an der anderen Griffbranche ein in einer Endstellung das zylindrische oder kugelige Ende des Betätigungselementes teilweise umgreifenden Rückhalteorgan gelagert ist, das in eine andere Endstellung bewegbar ist, in der das zylindrische oder kugelige Ende in Längsrichtung von der anderen Griffbranche entfernbar ist.

Bei Rohrschaftinstrumenten dieser Art (GB-2 140 735 A), die sehr vielfältig eingesetzt werden können, ist es wichtig, zum Zwecke des Werkzeugaustausches und zum Zwecke der Reinigung das Instrument möglichst vollständig zerlegen zu können.

Es sind Instrumente dieser Art bekannt, bei denen die Griffbranchen über Gewindeverbindungen mit dem Rohr und mit dem Betätigungselement verbunden werden können. Die Herstellung und Lösung der Verbindung dauert relativ lange, da in der Regel Feingewinde verwendet werden müssen, die eine große Zahl von Umdrehungen benötigen. Außerdem ist es schwierig, die konzentrischen Teile gleichzeitig in dieser Weise zu verbinden, da zwei verschiedene Verbindungen hergestellt werden müssen.

Chirurgische Instrumente der eingangs beschriebenen Art werden bereits angeboten (AESCULAP Hauptkatalog, Ausgabe 1991, Seite 647). Es ist jedoch notwendig, zur Festlegung ein separates, radial abstehendes Teil vorzusehen, welches das Herstellen und Lösen der Verbindung unter gewissen Umständen kompliziert gestalten kann.

Es ist Aufgabe der Erfindung, ein gattungsgemäßes Instrument so auszubilden, daß das Herstellen und Lösen der Verbindung zwischen Rohr und Betätigungselement einerseits und den Griffbranchen andererseits erleichtert wird, außerdem soll auch eine Ver- und Entriegelung der lösbaren Lagerung des Werkzeuges erleichtert werden.

Diese Aufgabe wird bei einem chirurgischen Instrument der eingangs beschriebenen Art erfindungsgemäß dadurch gelöst, daß der Verriegelungskörper eine Kugel ist, daß die radiale Bewegung des Verriegelungskörpers durch einen an der Außenseite des Verriegelungskörpers anliegenden Anschlag begrenzt ist, der durch eine axial auf der rohrförmigen Aufnahme verschiebliche Hülse gebildet wird, daß die Hülse relativ zu dem Verriegelungskörper derart verschiebbar ist, daß der Verriegelungskörper unterschiedlich weit radial nach Verriegelungskörper unterschiedlich weit radial nach außen verschiebbar ist, daß die Hülse einen Anschlag für ein auf dem Rohr axial verschiebbar gelagertes Außenrohr bildet, das in der vorgeschobenen Endstellung die lösbare Lagerung des Werkzeuges am gegenüberliegenden Ende des Rohres verriegelt, und daß die Hülse zur Freigabe der Verriegelung des Rohres in Richtung auf die Griffbranchen verschiebbar ist.

Es werden also sowohl für das Rohr als auch für das Betätigungselement getrennte, verschiebbare Verschlußelemente verwendet, die jeweils durch eine Bewegung zwischen einer Schließstellung und einer Öffnungsstellung verschoben oder verdreht werden können, so daß die Lösung und Verbindung sich sehr einfach gestalten. Insbesondere lassen sich diese Vorgänge sehr schnell bewerkstelligen.

Die Ausgestaltung der Verriegelung des Rohres in der einen Branche als Kugelgesperre erleichtert die Herstellung und auch die Lösung dieser Verbindung ganz erheblich. Zwar sind Kugelgesperre als Schnellkupplung für chirurgische Instrumente an sich bekannt (WO 91/05514), jedoch werden diese bekannten Schnellkupplungen im wesentlichen eingesetzt, um chirurgische Instrumente mit Halterungen oder Verlängerungen zu verbinden. Es ist aus der erwähnten Druckschrift nicht bekannt, ein Kugelgesperre zu verwenden, um den Schaft eines Rohrschaftinstrumentes mit einem Griffteil zu verbinden.

Zum Lösen des Rohres von der Aufnahme genügt es, die Hülse auf der rohrförmigen Aufnahme in eine Endstellung zu verschieben, dann kann der Verriegelungskörper in radialer Richtung das Rohr freigeben. Die auf der rohrförmigen Aufnahme verschiebbare Hülse fixiert dabei nicht nur den Verriegelungskörper, der das Rohr in der rohrförmigen Aufnahme festlegt, sondern außerdem noch ein Außenrohr auf dem Rohr, das seinerseits am vorderen Ende des Rohres eine Verriegelung für ein auswechselbares Werkzeug bildet.

Vorteilhaft ist es, wenn die Hülse federbelastet in die Endstellung verschoben wird, in der der Verschiebeweg des Verriegelungskörpers am stärksten eingeschränkt ist. Dadurch verschiebt die Feder die Hülse immer in die Verriegelungsstellung, nur entgegen der Kraft dieser Feder ist eine Lösung der axialen Festlegung des Rohres in der Aufnahme möglich.

Bei einer anderen Ausführungsform kann vorgesehen sein, daß die rohrförmige Aufnahme einen Anschlag für ein auf dem Rohr axial verschiebbar gelagertes Außenrohr bildet, das in der vorgeschobenen Endstellung die lösbare Lagerung des Werkzeuges an dem gegenüberliegenden Ende des Rohres verriegelt. Bei dieser Ausführung kann das Außenrohr auf dem Rohr nur verschoben werden, wenn das Rohr aus der rohrförmigen Aufnahme herausgezogen ist, d.h. ein Auswechseln des Werkzeuges setzt zunächst eine Trennung des gesamten Instrumentes voraus.

Günstig ist es, wenn das Rohr durch Eingreifen von Vor- bzw. Rücksprüngen beim Einschieben in die rohrförmige Aufnahme gegenüber einer Drehung um die Längsachse des Rohres gesichert ist. Damit läßt sich eine definierte Lage der Werkzeugebene erreichen. Es kann vorgesehen sein, daß Vor- und Rücksprünge dabei in unterschiedlichen Winkelstellungen eingreifen können, der Operateur kann also wahlweise durch kurzzeitige Trennung des Rohres vom Instrument dieses in unterschiedliche Winkelstellungen drehen und in diesen festlegen.

Bei einer abgewandelten Ausführungsform kann auch vorgesehen sein, daß die rohrförmige Aufnahme um ihre Längsachse drehbar an der Griffbranche gelagert ist. Durch Drehung dieser rohrförmigen Aufnahme kann dabei das Rohr mit dem daran gelagerten Werkzeug in die gewünschte Lage verdreht werden, so daß der Operateur vollständig frei ist in der Winkelstellung des Werkzeuges.

Bei einem ersten bevorzugten Ausführungsbeispiel kann vorgesehen sein, daß das Rückhalteorgan als Spannzange ausgebildet ist, die in gelöstem Zustand unter elastischer Aufbiegung den Ein- oder Austritt des kugelförmigen oder zylindrischen Endes ermöglicht und dieses in geschlossenem Zustand unlösbar umgreift. Eine solche Spannzange läßt sich beispielsweise durch axiales Einschieben in eine Hülse verriegeln, dieses Einschieben kann gegebenenfalls durch eine Gewindemutter vorgenommen werden.

Bei einer anderen Ausführungsform ist vorgesehen, daß das Rückhalteorgan schwenkbar an der anderen Griffbranche gelagert ist und im Schließzustand in einen an das kugelige oder zylindrische Ende anschließenden Abschnitt des Betätigungselementes eingreift, dessen Außenabmessungen quer zur Längsrichtung des Betätigungselementes kleiner sind als die des kugeligen oder zylindrischen Endes. Es genügt also zum Lösen der Drehverbindung zwischen Betätigungselement einerseits und Griffbranche andererseits, das Rückhalteorgan an der Griffbranche zu verschwenken, d.h. es genügt ein Handgriff, um wahlweise den Schließzustand oder den Freigabezustand zu erreichen.

Besonders vorteilhaft ist es, wenn das Rückhalteorgan einen Aufnahmeraum für das kugelige oder zylindrische Ende mit einer Öffnung aufweist, die einen größeren, für den Durchtritt des kugeligen oder zylindrischen Endes ausreichenden Bereich und einen kleineren, für dessen Durchtritt zu engen Bereich umfaßt, und daß die beiden Bereiche durch Bewegung des Rückhalteorgans wahlweise in Längsrichtung des Betätigungselementes vor dessen kugeliges oder zylindrisches Ende bewegbar sind. Der Aufnahmeraum umschließt das kugelige oder zylindrische Ende und überträgt dadurch zuverlässig sowohl Zug- als auch Druckbewegungen. Allein durch Verschwenken des Rückhalteorganes wird es möglich, das Betätigungselement freizugeben, dann nämlich, wenn der größere Bereich der Öffnung in Längsrichtung des Betätigungselementes gerichtet wird.

Günstig ist es, wenn das Betätigungsorgan in seinen Endstellungen durch elastische Rasten positionierbar ist.

Die Öffnung des Aufnahmeraumes kann insbesondere schlüssellochförmig sein.

Die nachfolgende Beschreibung bevorzugter Ausführungsformen der Erfindung dient im Zusammenhang mit der Zeichnung der näheren Erläuterung. Es zeigen:

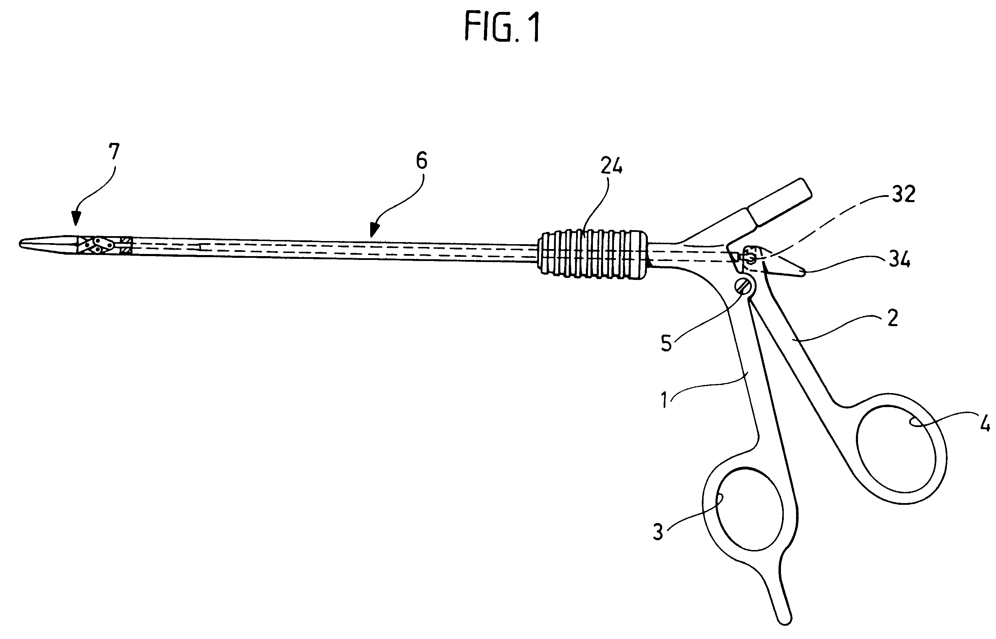

- Fig. 1: Eine Seitenansicht eines zerlegbaren Rohrschaftinstrumentes;
- Fig. 2: eine Längsschnittansicht des vorderen Teils und des mittleren Teils des Rohrschaftinstrumentes der Fig. 1;

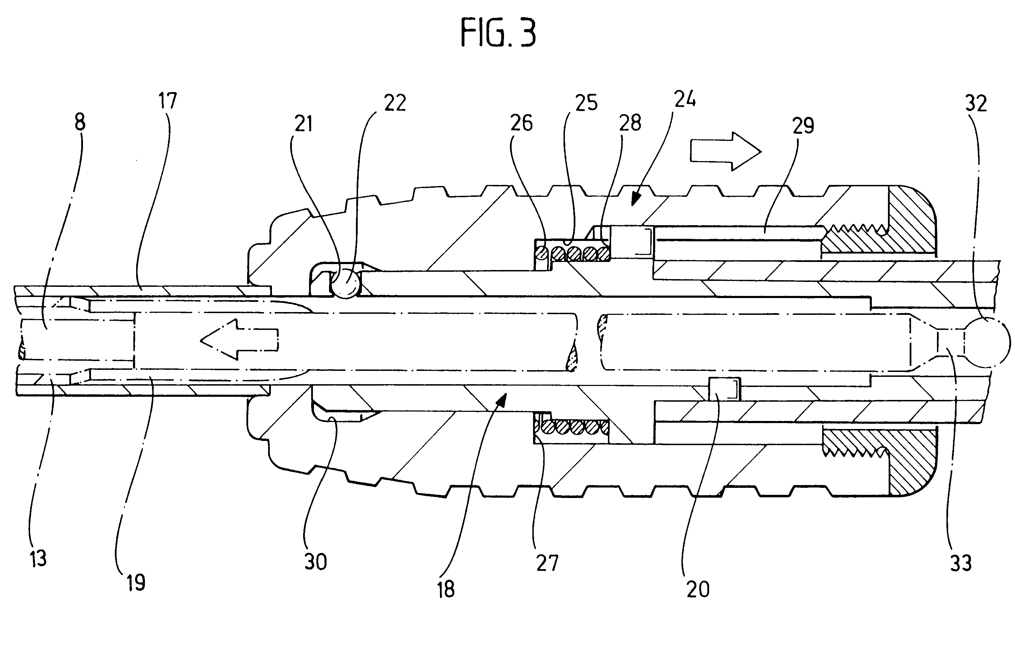

- Fig. 3: eine vergrößerte Längsschnittansicht des mittleren Teils des Rohrschaftinstrumentes der Fig. 1 im entriegelten Zustand;
- Fig. 4: eine Längsschnittansicht des Rohrschaftinstrumentes der Fig. 1 im Bereich der lösbaren Lagerung des Betätigungselementes an der Griffbranche mit verriegeltem Rückhalteorgan;
- Fig. 5: eine Ansicht ähnlich Fig. 4 mit geöffnetem Rückhalteorgan;
- Fig. 6: eine Ansicht ähnlich Fig. 4 bei einem abgewandelten Ausführungsbeispiel eines Rückhalteorgans in geschlossenem Zustand;
- Fig. 7: eine Ansicht des Rückhalteorgans in Richtung des Pfeiles A in Fig. 6;
- Fig. 8: eine Ansicht ähnlich Fig. 4 bei einem als Spannzange ausgebildeten Rückhalteorgan in geschlossenem Zustand;
- Fig. 9: eine Ansicht ähnlich Fig. 8 mit der Spannzange in geöffnetem Zustand;
- Fig. 10: eine Ansicht ähnlich Fig. 4 bei einem abgewandelten Ausführungsbeispiel eines verschwenkbaren Rückhalteorgans und
- Fig. 11: eine Schnittansicht längs Linie 11-11 in Fig. 10.

Das in der Zeichnung dargestellte Rohrschaftinstrument umfaßt zwei schwenkbar miteinander verbundene Griffbranchen 1 und 2 mit je einer Fingeröffnung 3 bzw. 4. Die beiden Griffbranchen 1 und 2 sind durch eine Schraubverbindung 5 schwenkbar miteinander verbunden. An einer Griffbranche 1 ist ein Schaft 6 gehalten, an dessen freiem Ende sich ein Werkzeug 7 befindet, das beispielsweise die Form einer Schere oder einer Zange haben kann. Im Inneren des Schaftes 6 ist ein stangenförmiges Betätigungselement 8 längsverschieblich angeordnet, das einerseits mit dem Werkzeug 7 und andererseits mit der anderen Griffbranche 2 gelenkig verbunden ist, so daß beim Öffnen und Schließen der Griffbranchen 1 und 2 durch eine Zug- oder Druckbewegung des Betätigungselementes 8 das Werkzeug 7 geöffnet und geschlossen werden kann.

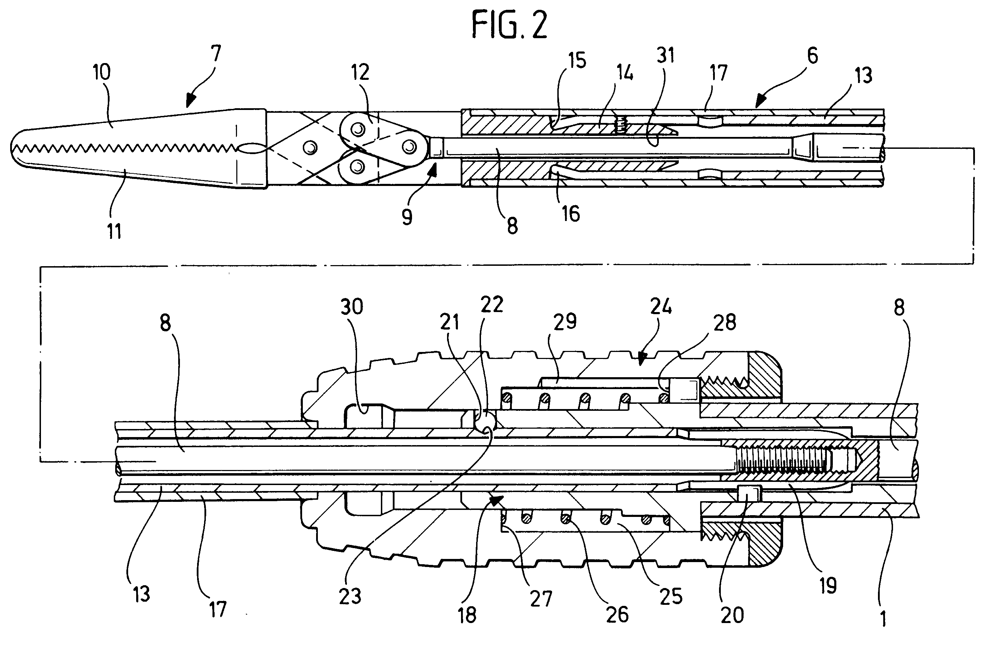

Dieses Werkzeug 7 umfaßt im dargestellten Ausführungsbeispiel 2 an einer Halterung 9 drehbar gelagerte Teile 10, 11, die über Getriebemittel 12 mit dem Betätigungselement 8 schwenkbar verbunden sind (Fig. 2). Diese Halterung 9 ist lösbar mit einem Rohr 13 verbunden. Zu diesem Zweck trägt die Halterung 9 eine in das Rohr 13 eintauchende Verlängerung 14 mit einer Umfangsnut 15. Das Rohr 13 ist an seinem freien Ende durch Längsschlitze in elastisch aufbiegbare Zungen 16 unterteilt, die an ihrem freien Ende nach innen abgebogen sind. Diese Zungen 16 umgeben die Verlängerung 14 und tauchen mit dem abgebogenen Ende in die Umfangsnut 15 ein, so daß die Verlängerung 14 im Rohr 6 in axialer Richtung festgelegt wird. In dieser Position werden die Zungen 16 dadurch dauerhaft gehalten, daß ein auf dem Rohr 13 längsverschiebliches Außenrohr 17 über die Zungen 16 geschoben ist, so daß die Zungen 16 an einer radial nach außen gerichteten Bewegung gehindert sind. Das Außenrohr 17 bildet somit eine Verriegelung, die nur gelöst werden kann, wenn das Außenrohr 17 über die Zungen 16 zurückgeschoben wird.

Das Rohr 13 und das darauf verschieblich gelagerte Außenrohr 17 bilden zusammen den Schaft 6, der lösbar mit der Griffbranche 1 verbunden werden kann. Dazu trägt diese Griffbranche 1 eine rohrförmige Aufnahme 18, die gegebenenfalls relativ zur Griffbranche 1 um die Längsachse der Aufnahme 18 verdrehbar sein kann. In diese Aufnahme 18 ist das Rohr 13 einschiebbar, der Außendurchmesser des Rohres 13 entspricht dem Innendurchmesser der Aufnahme 18. Das Rohr 13 weist an seinem in die Aufnahme 18 eingeschobenen Ende Längsschlitze 19 auf, in die ein in der rohrförmigen Aufnahme 18 gehaltener Vorsprung 20 hineinragt. Dadurch wird das Rohr 13 in der Aufnahme 18 unverdrehbar gelagert. Es können mehrere Längsschlitze vorgesehen sein, so daß das Rohr 13 in unterschiedlichen Winkelstellungen in der Aufnahme 18 festgelegt werden kann.

In einer Radialbohrung 21 der rohrförmigen Aufnahme 18 ist ein Verriegelungskörper 22 in Form einer Kugel gelagert, der bei in die Aufnahme 18 eingeschobenem Rohr 13 in eine Vertiefung 23 im Außenmantel des Rohres 13 eintaucht. In aus der Zeichnung nicht ersichtlicher Weise ist die Radialbohrung 21 auf der Innenseite nicht ganz durchgehend ausgeführt, so daß auch bei fehlendem Rohr 13 die Kugel 22 nicht radial nach innen aus der Radialbohrung 21 herausfallen kann. Nach außen ist die Radialbohrung 21 jedoch durchgehend ausgebildet, so daß die Kugel 22 radial nach außen frei bewegbar ist.

Auf der rohrförmigen Aufnahme 18 ist in Längsrichtung verschieblich eine die Aufnahme 18 umgebende Hülse 24 gelagert, die in einem zwischen der Aufnahme 18 und der Hülse 24 angeordneten Ringraum 25 eine Schraubenfeder 26 umgibt. Diese stützt sich einerseits an einer Stufe 27 der Hülse 24 und andererseits an einer Stufe 28 der Aufnahme 18 ab und beaufschlagt somit die Hülse 24 mit einer diese in Richtung des Werkzeuges 7 verschiebenden Kraft. Die Stufe 28 ist dabei in einer Längsnut 29 der Hülse 24 geführt, so daß dadurch die Hülse 24 gegen eine Verdrehung relativ zur Aufnahme 18 gesichert ist.

Die Bewegung der Hülse 24 in Richtung auf das Werkzeug 7 wird dadurch begrenzt, daß die Hülse 24 an dem Außenrohr 17 anschlägt und dieses somit in seine vorderste Stellung verschiebt, in der die Zungen 16 verriegelt sind (Fig. 2). Nur bei gegen die Wirkung der Schraubenfeder 26 zurückgeschobener Hülse 24 kann daher das Außenrohr 17 soweit zurückgeschoben werden, daß die Halterung 9 aus dem Rohr 13 herausgezogen werden kann.

Es ist auch möglich, daß die Hülse 24 mit dem Außenrohr 17 dauerhaft verbunden ist, dann ist ein Auswechseln des Werkzeuges 7 nur möglich, wenn das Rohr 13 aus der Aufnahme 18 herausgezogen worden ist.

Bei einem in der Zeichnung nicht dargestellten Ausführungsbeispiel könnte auch vorgesehen sein, daß das Außenrohr 17 an der Aufnahme 18 anschlägt, auch in diesem Falle wäre es zu einer Verschiebung des Außenrohres 17 notwendig, das Rohr 6 aus der Aufnahme 18 herauszuziehen. In diesem Falle könnte die Bewegung der Hülse 24 durch einen anderen geeigneten Anschlag begrenzt werden.

Die Hülse 24 liegt mit ihrer Innenwand an der Außenseite der Aufnahme 18 derart an, daß die Radialbohrung 21 verschlossen wird, so daß bei entspannter Schraubenfeder 26 die Bewegung der Kugel 22 radial nach außen verhindert wird. Die Abmessungen sind dabei so getroffen, daß in diesem Falle die Kugel 22 in die Vertiefung 23 eintaucht.

Wenn die Hülse 24 unter Zusammendrücken der Schraubenfeder 26 in eine Offenstellung verschoben wird, verschiebt sich eine Umfangsnut 30 an der Innenseite der Hülse 24 über die Radialbohrung 21, so daß dann die Kugel 22 in der Radialbohrung 21 in begrenztem Umfange radial nach außen verschoben werden kann, und zwar so weit, daß die Kugel 22 aus der Vertiefung 23 vollständig austritt. In dieser Position ist die durch die Kugel 22 erzeugte axiale Festlegung des Rohres 13 in der Aufnahme 18 aufgehoben, das Rohr 13 kann daher in dieser Position aus der Aufnahme 18 herausgezogen werden.

Das stangenförmige Betätigungselement 8 ragt durch eine zentrale Bohrung 31 der Verlängerung 14 hindurch und steht nach hinten über das Rohr 13 über, und zwar so weit, daß es auch aus der Griffbranche 1 heraussteht. In diesem Bereich trägt das stangenförmige Betätigungselement 8 ein kugeliges Ende 32, das über einen Verbindungsabschnitt 33 mit einem geringeren Außendurchmesser mit dem Betätigungselement 8 verbunden ist.

Das kugelige Ende 32 bildet eine Gelenkverbindung mit der zweiten Griffbranche 2.

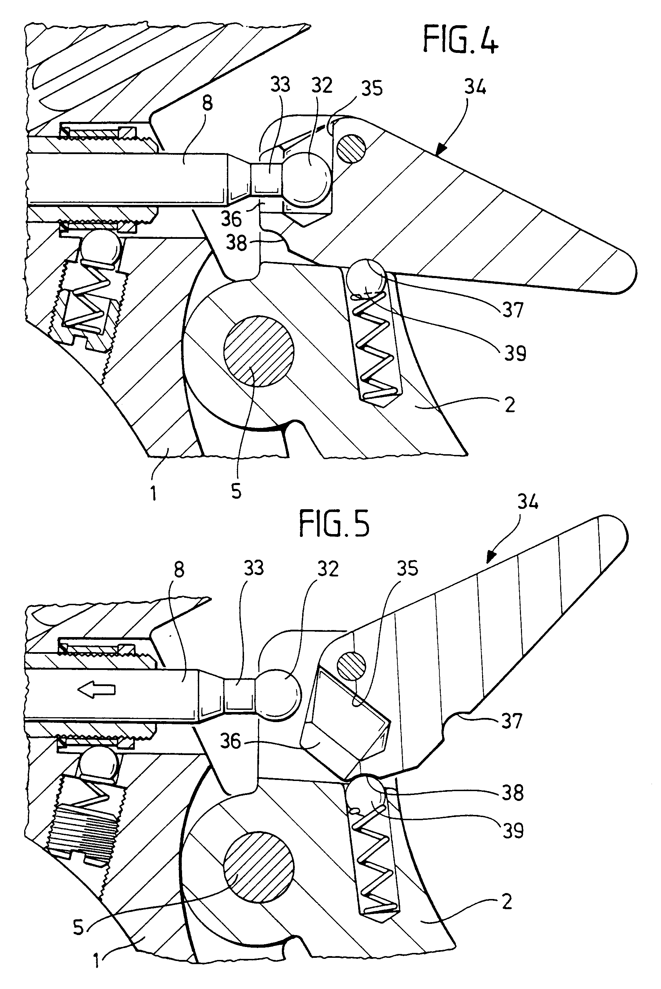

Zu diesem Zweck ist bei einem in Fig. 4 und 5 beschriebenen ersten Ausführungsbeispiel an der Griffbranche 2 um eine quer zur Längsrichtung des Betätigungselementes 8 verlaufende horizontale Achse ein Schwenkhebel 34 verschwenkbar gelagert, der eine einseitig offene Sacklochbohrung 35 aufweist. Diese steht über einen parallel zu ihrer Längsrichtung verlaufenden Längsschlitz 36 mit der Umgebung in Verbindung. Der Durchmesser der Sacklochbohrung 35 entspricht im wesentlichen dem Außendurchmesser des kugeligen Endes 32, die Breite des Längsschlitzes 36 ist kleiner gewählt und entspricht im wesentlichen dem Durchmesser des Betätigungselementes 8 im schmaleren Verbindungsabschnitt 33. Der Schwenkhebel 34 ist zwischen einer Schließstellung (Fig. 4) und einer Offenstellung (Fig. 5) verschwenkbar und wird in beiden Endstellungen durch eine federnd in Ausnehmungen 37 bzw. 38 eingreifende Kugel 39 festgelegt. In der Schließstellung wird das kugelige Ende 32 in der Sacklochbohrung 35 aufgenommen, diese Sacklochbohrung 35 bildet also einen Aufnahmeraum für das kugelige Ende 32. In dieser Position des Schwenkhebels 34 ist die Sacklochbohrung 35 nach oben geöffnet, also senkrecht zur Längsrichtung des Betätigungselementes 8, so daß ein Herausziehen des Betätigungselementes zur Vorderseite des Instrumentes hin unmöglich ist.

In der in Fig. 5 dargestellten Öffnungsstellung zeigt das offene Ende der Sacklochbohrung 35 in Richtung des Betätigungselementes 8, so daß dieses in Richtung des in Fig. 5 angebenen Pfeiles aus dem Instrument herausgezogen werden kann. Die Festlegung des Betätigungselementes 8 erfolgt also bei diesem Ausführungsbeispiel allein durch Verschwenken des Schwenkhebels 34, der somit die Funktion eines Rückhalteorgans für das Betätigungselement 8 erhält.

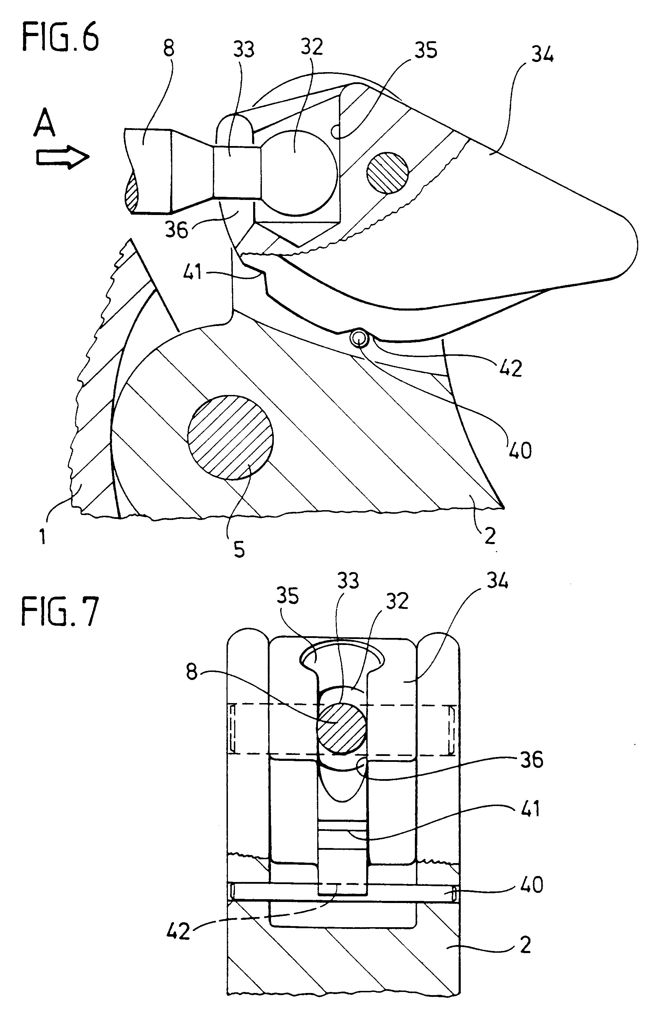

Im Ausführungsbeispiel der Fig. 6 und 7 ist eine sehr ähnliche Lösung dargestellt, gleiche Teile tragen daher dieselben Bezugszeichen. Der Schwenkhebel 34 wird in den Endstellungen in diesem Falle nicht durch eine federbelastende Kugel festgelegt, sondern durch einen Haltestift 40, der federnd in Kerben 41, 42 des Schwenkhebels 34 einrastet. Die Elastizität des für den Schwenkhebel verwendeten Materials kann hier ausreichen, um die notwendigen Verschiebungen zu ermöglichen.

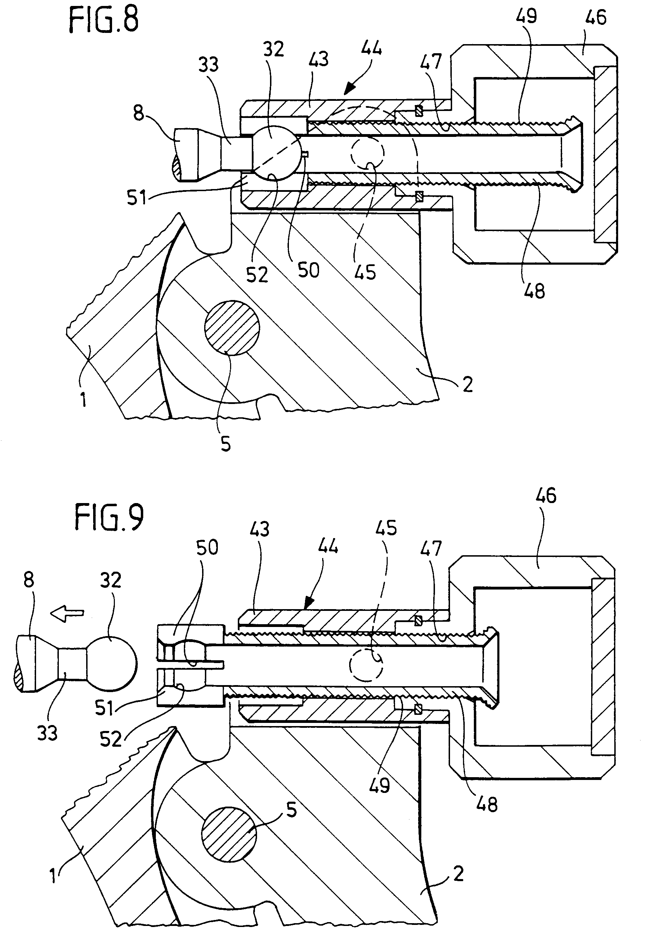

Bei dem Ausführungsbeispiel der Fig. 8 und 9 ist an der Griffbranche 2 eine Spannzange 44 um eine quer zur Längsachse des Betätigungselementes 8 angeordnete horizontale Achse 45 verschwenkbar gelagert. Diese Spannzange 44 umfaßt eine Lagerhülse 43 und eine axial unverschieblich und drehbar in dieser gelagerte Innengewindehülse 47 mit einem Griffteil 46. In der Innengewindehülse 47 ist ein Rohrstück 48 in einem Außengewinde 49 eingeschraubt, welches bei Verdrehung des Griffteiles 46 mehr oder weniger tief in die Lagerhülse 43 eingezogen wird. Am freien Ende des Rohrstückes 48 sind durch Längsschlitze 50 elastisch radial nach außen biegbare Bereiche 51 ausgebildet, die einen kugelkalottenförmigen Aufnahmeraum 52 umgeben, dessen Abmessungen den Außenabmessungen des kugeligen Endes 32 des Betätigungselementes 8 entsprechen. Unter elastischem Aufbiegen der Bereiche 51 kann das kugelige Ende 32 in den kugelkalottenförmigen Aufnahmeraum 52 eingeschoben werden, so daß die Bereiche 51 das kugelige Ende 32 teilweise gelenkig umgreifen.

Diese Verbindung ist jederzeit wieder lösbar, solange die Bereiche 51 elastisch nach außen abbiegbar sind, wie dies in Fig. 9 dargestellt ist. Durch Verdrehen des Griffteiles 46 kann jedoch die Innengewindehülse 47 so weit in die Lagerhülse 43 eingezogen werden, daß die elastischen Bereiche 51 an der Innenseite der Lagerhülse 43 anliegen und dadurch an einem elastischen Verformen nach außen gehindert werden. Dies ist in Fig. 8 dargestellt. Dadurch wird das kugelige Ende 32 im Aufnahmeraum 52 unlösbar, jedoch gelenkig festgelegt. Zum Lösen dieser Verbindung genügt es, durch Verdrehung des Griffteiles 46 die Innengewindehülse 47 wieder so weit aus der Lagerhülse 43 herauszuschieben, bis die elastischen Bereiche 51 aufbiegbar sind.

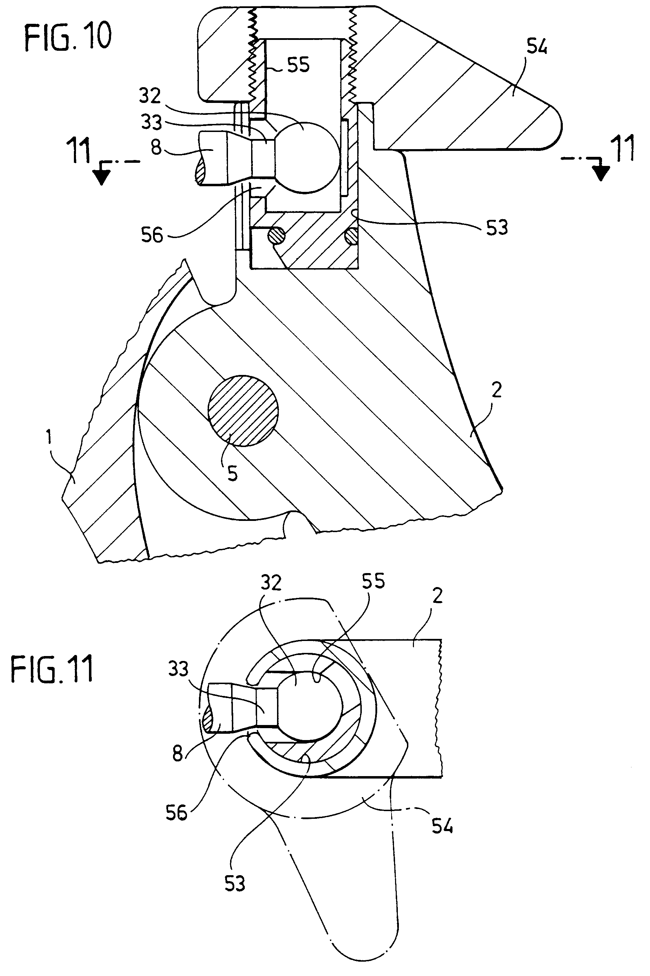

Im Ausführungsbeispiel der Fig. 10 und 11 ist in einer senkrechten Bohrung 53 der Griffbranche 2 ein Schwenkkörper 54 eingesetzt, der um die senkrechte Achse der Bohrung 53 verdrehbar ist. Im Schwenkkörper 54 ist koaxial zur Bohrung 53 eine Sacklochbohrung 55 angeordnet, die über eine querliegende, schlüssellochförmige Öffnung 56 mit dem Außenraum in Verbindung steht. In einer ersten Position ist der enge Bereich der schlüssellochförmigen Öffnung 56 in Längsrichtung des Betätigungselementes 8 angeordnet, so daß ein Austritt des größeren kugeligen Endes 32 verhindert wird, in der anderen Endposition befindet sich jedoch der größere Teil der Schlüssellochförmigen Öffnung 56 in dieser Richtung, so daß in dieser Position das kugelige Ende 32 aus der Öffnung 56 austreten kann. Auch bei diesem Ausführungsbeispiel könnte der in diesem Falle um die senkrechte Achse verdrehbare Schwenkkörper 54 durch Rasten in den Endstellungen festgelegt sein, dies ist in der Zeichnung nicht extra dargestellt.

Bei der Montage des beschriebenen Instrumentes wird in jedem Falle zuerst die Halterung 9 mit dem Werkzeug 7 in das vordere Ende des Rohres 13 eingeschoben, bis die Zungen 16 in die Umfangsnut 15 einrasten. Das dauerhaft mit dem Werkzeug 7 verbundene Betätigungselement 8 wird dabei durch die Bohrung 31 hindurchgeführt, und das freie Ende des Betätigungselementes 8 steht an der rückwärtigen Seite des Rohres 13 über dieses hervor.

Zur Festlegung der Verbindung zwischen Werkzeug 7 und Rohr 13 kann das Außenrohr 17 so verschoben werden, daß es die Zungen 16 überdeckt. Diese Einheit wird daraufhin in die rohrförmige Aufnahme 18 eingeschoben, wobei gleichzeitig die Hülse 24 entgegen der Wirkung der Schraubenfeder 26 verschoben wird. Dadurch ist ein ungehindertes Eintreten des Rohres 13 in die Aufnahme 18 möglich, ohne daß die Kugel 22 diese Eintrittsbewegung verhindert. Beim Einschieben des Rohres 13 tritt der Vorsprung 20 in einen der Längsschlitze 19 ein und richtet dadurch das Rohr in Umfangsrichtung in die gewünschte Winkelstellung aus. Sobald die gewünschte Einschubtiefe erreicht ist, wird die Hülse 24 losgelassen, so daß sie sich unter der Wirkung der Schraubenfeder 26 in die Schließstellung verschiebt und dabei die Kugel 22 in die ausgerichtete Vertiefung 23 des Rohres 13 einschiebt. Dadurch ist das Rohr 13 in der Aufnahme in axialer Richtung festgelegt. Dadurch ergibt sich auch eine Fixierung des Außenrohres 17 auf dem Rohr 13, so daß nunmehr auch das Werkzeug 7 im Rohr 6 dauerhaft verriegelt ist.

Das kugelige Ende 32 des Betätigungselementes 8 ist bei dieser Einschubbewegung des Rohres 13 in das in Offenstellung stehende Rückhalteorgan eingetreten. Durch Bewegung des Rückhalteorganes in die Schließstellung wird das kugelige Ende 32 von dem Rückhalteorgan teiweise gelenkig umgeben und außerdem wird verhindert, daß das Ende 32 in Richtung des Betätigungselementes 8 verschoben werden kann. Dadurch lassen sich Zug- und Druckkräfte von der Griffbranche 2 auf das Betätigungselement 8 übertragen.

Zum Zerlegen des Instrumentes wird in umgekehrter Reihenfolge vorgegangen.

## Patentansprüche

1. Chirurgisches Rohrschaftinstrument mit zwei gelenkig miteinander verbundenen Griffbranchen (1, 2), mit einem an einer Griffbranche (1) gehaltenen Rohr (13), einem an dessen Ende lösbar gelagerten Werkzeug (7), einem im Rohr (13) längsverschieblich gelagerten, stabförmigen Betätigungselement (8) für das Werkzeug (7), das über ein zylindrisches oder kugeliges Ende (32) gelenkig mit der anderen Griffbranche (2) verbunden ist, und mit einer lösbaren Verbindung zwischen Rohr (13) und Betätigungselement (8) einerseits und den beiden Griffbranchen (1, 2) andererseits, wobei das Rohr (13) in eine rohrförmige Aufnahme (18) an der einen Griffbranche (1) einschiebbar und in dieser durch mindestens einen radial bewegbaren, in einer Endstellung mit dem Rohr (13) einen Formschluß eingehenden Verriegelungskörper (22) axial festlegbar ist und wobei an der anderen Griffbranche (2) ein in einer Endstellung das zylindrische oder kugelige Ende (32) des Betätigungselementes (8) teilweise umgreifendes Rückhalteorgan (34, 44, 54) gelagert ist, das in eine andere Endstellung bewegbar ist, in der das zylindrische oder kugelige Ende (32) in Längsrichtung von der anderen Griffbranche (2) entfernbar ist,
**dadurch gekennzeichnet**, daß der Verriegelungskörper (22) eine Kugel ist, daß die radiale Bewegung des Verriegelungskörpers (22) durch einen an der Außenseite des Verriegelungskörpers (22) anliegenden Anschlag begrenzt ist, der durch eine axial auf der rohrförmigen Aufnahme (18) verschiebliche Hülse (24) gebildet wird, daß die Hülse (24) relativ zu dem Verriegelungskörper (22) derart verschiebbar ist, daß der Verriegelungskörper (22) unterschiedlich weit radial nach außen verschiebbar ist, daß die Hülse (24) einen Anschlag für ein auf dem Rohr (13) axial verschiebbar gelagertes Außenrohr (17) bildet, das in der vorgeschobenen Endstellung die lösbare Lagerung des Werkzeuges (7) am gegenüberliegenden Ende des Rohres (13) verriegelt, und daß die Hülse (24) zur Freigabe der Verriegelung des Rohres (13) in Richtung auf die Griffbranchen (1, 2) verschiebbar ist.

2. Instrument nach Anspruch 1,
dadurch gekennzeichnet, daß die Hülse (24) federbelastet in die Endstellung verschoben wird, in der der Verschiebeweg des Verriegelungskörpers (22) am stärksten eingeschränkt ist.

3. Instrument nach einem der Ansprüche 1 oder 2,
dadurch gekennzeichnet, daß die rohrförmige Aufnahme (18) einen Anschlag für ein auf dem Rohr (13) axial verschiebbar gelagertes Außenrohr (17) bildet, das in der vorgeschobenen Endstellung die lösbare Lagerung des Werkzeuges (7) am gegenüberliegenden Ende des Rohres (13) verriegelt.

4. Instrument nach einem der voranstehenden Ansprüche,
dadurch gekennzeichnet, daß das Rohr (13) durch Eingreifen von Vor- bzw. Rücksprüngen (19, 20) beim Einschieben in die rohrförmige Aufnahme (18) gegenüber einer Drehung um die Längsachse des Rohres (13) gesichert ist.

5. Instrument nach Anspruch 4,
dadurch gekennzeichnet, daß Vor- und Rücksprünge (19, 20) in unterschiedlichen Winkelstellungen eingreifen können.

6. Instrument nach einem der voranstehenden Ansprüche,
dadurch gekennzeichnet, daß die rohrförmige Aufnahme (18) um ihre Längsachse drehbar an der Griffbranche (1) gelagert ist.

7. Instrument nach einem der voranstehenden Ansprüche,
dadurch gekennzeichnet, daß das Rückhalteorgan als Spannzange (44) ausgebildet ist, die in gelöstem Zustand unter elastischer Aufbiegung den Ein- oder Austritt des kugeligen oder zylindrischen Endes (32) ermöglicht und dieses im geschlossenen Zustand unlösbar umgreift.

8. Instrument nach einem der Ansprüche 1 bis 6,
dadurch gekennzeichnet, daß das Rückhalteorgan (34, 54) schwenkbar an der anderen Griffbranche (2) gelagert ist und im Schließzustand in einen an das kugelige oder zylindrische Ende (32) anschließenden Abschnitt (33) des Betätigungselementes (8) eingreift, dessen Außenabmessungen quer zur Längsrichtung des Betätigungselementes (8) kleiner sind als die des kugeligen oder zylindrischen Endes (32).

9. Instrument nach Anspruch 8,
dadurch gekennzeichnet, daß das Rückhalteorgan (34, 54) einen Aufnahmeraum (35, 55) für das kugelige oder zylindrische Ende (32) mit einer Öffnung (35, 36; 56) aufweist, die einen größeren, für den Durchtritt des kugeligen oder zylindrischen Endes (32) ausreichenden Bereich und einen kleineren, für dessen Durchtritt zu engen Bereich umfaßt, und daß die beiden Bereiche durch Bewegung des Rückhalteorgans (34, 54) wahlweise in Längsrichtung des Betätigungselementes (8) vor dessen kugeliges oder zylindrisches Ende (32) bewegbar sind.

10. Instrument nach Anspruch 9,
dadurch gekennzeichnet, daß das Rückhalteorgan (34, 54) in seinen Endstellungen durch elastische Rasten (39, 40) positionierbar ist.

11. Instrument nach einem der Ansprüche 9 oder 10,
dadurch gekennzeichnet, daß die Öffnung (56) schlüssellochförmig ausgebildet ist.

## Claims

1. A tubular-shaft surgical instrument comprising two gripping arms (1, 2) interconnected in an articulated manner, a tube (13) held on one gripping arm (1), an implement (7) detachably mounted at the end of the tube (13), a rod-shaped actuating member (8) for the implement (7), the actuating member (8) being longitudinally displaceably mounted in the tube (13) and being connected in an articulated manner to the other gripping arm (2) via a cylindrical or spherical end (32), and a detachable connection between the tube (13) and the actuating member (8) on the one hand and the two gripping arms (1, 2) on the other, wherein the tube (13) is insertable into a tubular mounting (18) in one gripping arm (1) and is axially securable therein by means of at least one radially movable locking member (22) producing a positive connection with the tube (13) in one end position, and wherein a retaining member (34, 44, 54), partly surrounding the cylindrical or spherical end (32) of the actuating member (8) in one end position, is mounted on the other gripping arm (2) and is movable into a second end position in which the cylindrical or spherical end (32) is removable from the other gripping arm (2) in the longitudinal direction, characterised in that the locking member (22) is a ball, in that the radial movement of the locking member (22) is limited by a stop resting against the outside of the locking member (22) and formed by a sleeve (24) axially displaceable on the tubular mounting (18), in that the sleeve (24) is displaceable relative to the locking member (22) in such a manner that the locking member (22) is displaceable radially outwards by varying amounts, in that the sleeve (24) forms a stop for an outer tube (17) which is axially displaceably mounted on the tube (13) and, in the advanced end position, locks the detachable mounting of the implement (7) at the opposite end of the tube (13), and in that the sleeve (24) is displaceable towards the gripping arms (1, 2) in order to release the locking of the tube (13).

2. An instrument according to claim 1, characterised in that the sleeve (24) is displaced under spring load into the end position in which the displacement path of the locking member (22) is most greatly restricted.

3. An instrument according to either one of claims 1 and 2, characterised in that the tubular mounting (18) forms a stop for an outer tube (17) which is axially displaceably mounted on the tube (13) and, in the advanced end position, locks the detachable mounting of the implement (7) at the opposite end of the tube (13).

4. An instrument according to any one of the preceding claims, characterised in that the tube (13) is secured against rotation about the longitudinal axis of the tube (13) owing to the engagement of projections and recesses (19, 20) when pushed into the tubular mounting (18).

5. An instrument according to claim 4, characterised in that projections and recesses (19, 20) can engage in various angular positions.

6. An instrument according to any one of the preceding claims, characterised in that the tubular mounting (18) is mounted on the gripping arm (1) so as to be rotatable about its longitudinal axis.

7. An instrument according to any one of the preceding claims, characterised in that the retaining member is formed as a collet (44) which, in the released state, allows the spherical or cylindrical end (32) to pass in and out by bending resiliently outwards and, in the locked state, engages non-detachably round the end (32).

8. An instrument according to any one of claims 1 to 6, characterised in that the retaining member (34, 54) is pivotably mounted on the other gripping arm (2) and, in the locked state, engages in a portion (33) of the actuating member (8) adjoining the spherical or cylindrical end (32), the outer dimensions of the portion (33) being smaller than those of the spherical or cylindrical end (32) transversely to the longitudinal direction of the actuating member (8).

9. An instrument according to claim 8, characterised in that the retaining member (34, 54) has a receiving chamber (35, 55) for the spherical or cylindrical end (32) and having an opening (35, 36; 56) comprising a larger region sufficient for the passage of the spherical or cylindrical end (32) and a smaller region too narrow for the passage thereof, and in that the two regions are movable by moving the retaining member (34, 54) as desired in the longitudinal direction of the actuating member (8) upstream of the spherical or cylindrical end (32) thereof.

10. An instrument according to claim 9, characterised in that the retaining member (34, 54) is positionable in its end positions by means of resilient detents (39, 40).

11. An instrument according to either one of claims 9 and 10, characterised in that the opening (56) is formed in the manner of a keyhole.

## Revendications

1. Instrument chirurgical à fût tubulaire comportant deux branches de prise (1, 2), reliées l'une à l'autre avec articulation, un tube (13) maintenu sur une branche de prise (1), un outil (7) porté, de façon détachable, à l'extrémité du tube, un élément de manoeuvre (8) en forme de tige, porté dans le tube (13) avec liberté de coulissement, pour l'outil (7), élément qui, par l'intermédiaire d'une extrémité cylindrique ou sphérique (32), est relié à l'autre branche de prise (2) avec articulation, et comportant une liaison détachable entre tube (13) et élément de manoeuvre (8) d'une part et les deux branches de prise (1, 2) d'autre part, dans lequel le tube (13) peut coulisser dans un réceptacle tubulaire (18) prévu sur la première branche de prise (1) et se bloquer axialement dans cette branche au moyen d'au moins un élément de verrouillage (22) qui peut se déplacer radialement et qui, dans une position d'extrémité, participe avec le tube (13) à une fixation par complémentarité de forme, et dans lequel, sur l'autre branche de prise (2), est porté un organe de retenue (34, 44, 54) qui, dans une position d'extrémité, enserre partiellement l'extrémité cylindrique ou sphérique (32) de l'élément de manoeuvre (8) et que l'on peut amener dans une autre position d'extrémité dans laquelle on peut, selon la direction longitudinale, séparer l'extrémité cylindrique ou sphérique (32) d'avec l'autre branche de prise (2),
caractérisé par le fait que l'élément de verrouillage (22) est une bille, que le déplacement radial de l'élément de verrouillage (22) est limité par une butée qui s'appuie contre la face extérieure de l'élément de verrouillage (22) et qui est formée par un manchon (24) qui peut coulisser axialement sur le réceptacle tubulaire (18), que le manchon (24) peut coulisser par rapport à l'élément de verrouillage (22) de façon que l'élément de verrouillage (22) puisse coulisser radialement plus loin vers l'extérieur, que le manchon (24) forme une butée pour un tube extérieur (17) qui peut coulisser axialement sur le tube (13) et qui, dans la position d'extrémité où il a coulissé vers l'avant, verrouille la portée amovible de l'outil (7) à l'extrémité opposée du tube (13), et que le manchon (24) peut coulisser en direction des branches de prise (1, 2) pour déverrouiller le verrouillage du tube (13).

2. Instrument selon la revendication 1,
caractérisé par le fait que, contraint par un ressort, le manchon (24) coulisse dans la position d'extrémité dans laquelle la course de coulissement de l'élément de verrouillage (22) est la plus limitée.

3. Instrument selon l'une des revendications 1 ou 2,
caractérisé par le fait que le réceptacle tubulaire (18) forme une butée pour un tube extérieur (17) qui est porté, avec liberté de coulissement axial, sur le tube (13) et qui, dans la position d'extrémité où il a coulissé vers l'avant, verrouille la portée amovible de l'outil (7) à l'extrémité opposée du tube (13).

4. Instrument selon l'une des revendications 1 ou 2,
caractérisé par le fait que le tube (13), lors de son coulissement dans le réceptacle tubulaire (18), est garanti à l'égard d'une rotation autour de l'axe longitudinal du tube (13) par venue en prise de saillies et d'évidements (19, 20).

5. Instrument selon la revendication 4,
caractérisé par le fait que des saillies et des évidements (19, 20) peuvent venir en prise dans des positions angulaires différentes.

6. Instrument selon l'une des revendications précédentes,
caractérisé par le fait que le réceptacle tubulaire (18) est porté sur la branche de prise (1) avec liberté de rotation autour de son propre axe longitudinal.

7. Instrument selon l'une des revendications précédentes,
caractérisé par le fait que l'organe de retenue est conçu sous forme de mors de serrage (44) qui, à l'état ouvert, permet, sous réserve d'un cintrage élastique, l'entrée ou la sortie de l'extrémité sphérique ou cylindrique (32) et, à l'état fermé, enserre celle-ci de façon imperdable.

8. Instrument selon l'une des revendications 1 à 6,
caractérisé par le fait que l'organe de retenue (34, 54) est porté sur l'autre branche de prise (2) avec liberté de pivotement et, à l'état fermé, vient en prise sur un tronçon (33) de l'élément de manoeuvre (8) qui se raccorde à l'extrémité sphérique ou cylindrique (32) et dont les dimensions extérieures, perpendiculairement à la direction longitudinale de l'élément de manoeuvre (8), sont inférieures à celles de l'extrémité sphérique ou cylindrique (32).

9. Instrument selon la revendication 8,
caractérisé par le fait que l'organe de retenue (34, 54) présente, pour l'extrémité sphérique ou cylindrique (32), un espace de réception (35, 55) avec une ouverture (35, 36; 56) qui comporte une zone plus grande, suffisante pour le passage de l'extrémité sphérique ou cylindrique (32), et une zone plus petite, trop étroite pour son passage, et que, par le mouvement de l'organe de retenue (34, 54), les deux zones peuvent être amenées au choix, selon la direction longitudinale de l'élément de manoeuvre (8), devant son extrémité sphérique ou devant son extrémité cylindrique (32).

10. Instrument selon la revendication 9,
caractérisé par le fait que l'organe de retenue (34, 54) peut être positionné dans ses positions d'extrémité par des crantages élastiques (39, 40).

11. Instrument selon l'une des revendications 9 ou 10,
caractérisé par le fait que l'ouverture (56) a la forme d'un trou de serrure.
